(19) Europäisches Patentamt
European Patent Office
Office européen des brevets

(11) Publication number: 0 171 414 B1

(12) **EUROPEAN PATENT SPECIFICATION**

(45) Date of publication of patent specification: 26.09.90

(51) Int. Cl.$^5$: C 07 C 239/16, C 07 C 239/22, A 61 K 31/16

(21) Application number: 85900906.0

(22) Date of filing: 24.01.85

(86) International application number: PCT/US85/00104

(87) International publication number: WO 85/03290 01.08.85 Gazette 85/17

(54) **ORALLY EFFECTIVE ION CHELATORS RELATED TO DEFEROXAMINE.**

(30) Priority: 26.01.84 US 574482

(43) Date of publication of application: 19.02.86 Bulletin 86/08

(45) Publication of the grant of the patent: 26.09.90 Bulletin 90/39

(84) Designated Contracting States: BE CH DE FR GB LI LU NL SE

(56) References cited: US-A-3 247 197

(73) Proprietor: ORAL-D
112 Fulton Street
Boston, MA 02109 (US)

(72) Inventor: GREEN, Donald, E.
765 Harvard Avenue
Sunnyvale, CA 94087 (US)

(74) Representative: Wuesthoff, Franz, Dr.-Ing. et al
Wuesthoff & Wuesthoff Patent- und Rechtsanwälte Schweigerstrasse 2
D-8000 München 90 (DE)

EP 0 171 414 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention is concerned with the preparation of compounds, compositions and methods which are useful for treating diseases in human beings which are a result of a body tri-valent ion (i.e. $Fe^{+++}$, $Al^{+++}$) overload state.

Iron overload diseases include thalassemia major, sideroachrestic anemia, Blackfan-Diamond anemia, aplastic anemia, sickle cell anemia, other hemolytic anemias, and a number of other diseases and conditions in which hemosiderosis (a focal or general increase in tissue iron stores without associated tissue damage) occurs. One type of hemosiderosis occurs in most patients after multiple blood transfusions have occurred. Another type of hemosiderosis occurs as the result of the treatment of an anemia found in kidney damaged patients where dialysis is used to remove toxic wastes. Treatment of these conditions has generally involved the administration of a chelating agent having a selective affinity for tissue $Fe^{+++}$ ion which can then be excreted as the iron chelate.

The ideal chelating agent for the reduction of tissue metal ions, e.g. iron, aluminum, gallium, ytterbium, indium and the like should have at least the following attributes:

1. Have high selectivity with respect to ion, e.g. iron, binding;
2. Be essentially metabolically inert;
3. Be essentially non-toxic;
4. Be inexpensive to produce; and
5. Be capable of oral administration.

Over the years a number of approaches have been investigated which have some of these attributes. The current drug of choice is deferoxamine, a compound obtained from the microorganism *streptomyces pilosus.* Deferoxamine has the following structure:

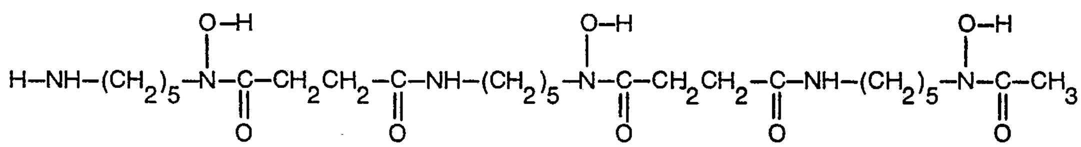

This material meets the aforementioned criteria except for oral availability. Deferoxamine (as the methanesulfonate salt) has been shown to be most effective when it is delivered parenterally via slow continuous (about an 8—12 hour period) subcutaneous infusion using a portable infusion pump, i.e., a battery powered syringe pump.

This administration route for iron overload conditions is particularly difficult in view of the widespread occurrence of the disease, thalassemia major, found in the population in countries bordering on the Mediterranean Sea and extending eastward through the Middle East, India to Southeast Asia, and in sickle cell anemia which is prevalent in the populations in Africa.

The present invention concerns certain acyl derivatives of deferoxamine which are effective ion, e.g. iron and aluminum chelators when administered orally.

Some compounds related to the compounds of the present invention are described in the literature by H. Bickel, et al. in *Helvetica Chimica Acta,* Vol. 46, No. 153, pp 1385—1389, published in 1963 and their related U.S. Patent No. 3,247,197.

The focus of these references is the preparation of N-acyl trihydroxy derivatives of deferoxamine which have the structure:

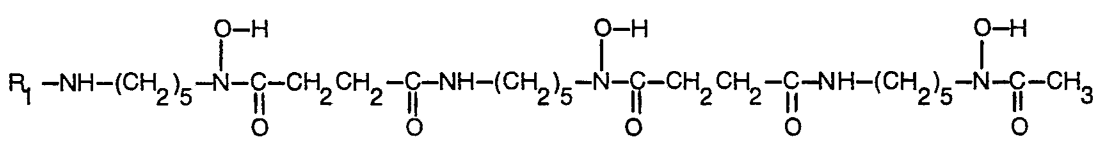

wherein $R_1$ may be an acyl group. These references mention tetra acyl materials, i.e., materials of the formula

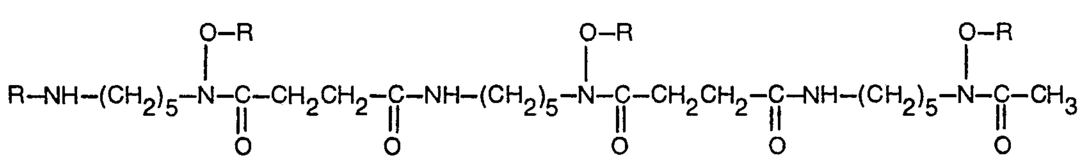

wherein the R groups are each acetyls, as intermediates in the production of their focus compounds. These references do not teach the use of tetra-acyl materials in body ion, e.g., iron, removal applications nor do they suggest that the tetra- or higher acyl materials either as pure isomers or as mixtures would be effective when orally administered in these applications.

U.S. Patents Nos. 3,118,823 and 3,153,621 are concerned with iron chelates of deferoxamine, which are used as growth factors. Additional references of interest in this art include Bickel, et. al., *Helvitica Chimica Acta,* Vol. 43, pp. 2118 ff and 2129 ff, published in 1960; and V. Prelog and Walser, *Helvitica Chimica Acta,*

Vol. 45, pp 631 ff, published in 1962. Finally, D. E. Green and T. B. Okarma briefly reported on studies on the preparation of some tetra-acyl derivatives of deferoxamine and the biological properties of these derivatives. (See Abstracts, 186th Annual American Chemical Society Meeting, August 28—September 2, 1983, Washington, D.C., Abstract No. MEDI 56.

The present invention concerns a group of di-, tri-, tetra-, penta-, hexa- and hepta-acylated derivatives of deferoxamine, which are useful in the treatment of the diseases or conditions cited earlier. The invention is particularly useful in that its compounds are orally administered, absorbed from the digestive system into the body and cleaved to produce deferoxamine in the body.

In one aspect, this invention relates to compounds of the general formula:

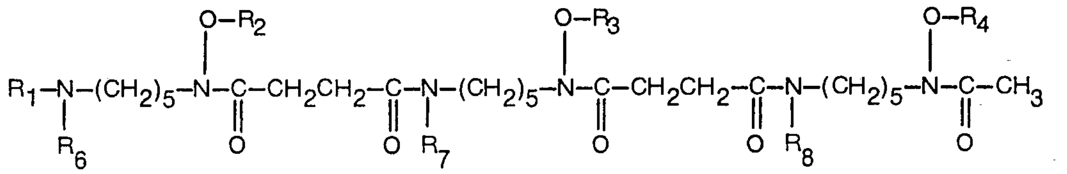

(I)

wherein:

$R_1$ is acyl of the formula —(C=O)—$R_5$; and

$R_2$, $R_3$, $R_4$, $R_6$, $R_7$ and $R_8$ are each independently selected from hydrogen, and acyl of the formula:

$$—C(=O)—R_5$$

wherein $R_5$ is selected from $C_{1-25}$ alkyls and substituted $C_1$—$C_{25}$ alkyls having 1 to 3 protons replaced by alkoxyl or halogen; such that at least one $R_2$, $R_3$ and $R_4$ is an acyl of the formula —C(=O)—$R_5$ different than $R_1$, when $R_6$, $R_7$ and $R_8$ are each hydrogen.

Compounds of formula I are prodrug forms of deferoxamine which liberate deferoxamine in the body to complex and/or chelate ions, such as iron and/or aluminum, for subsequent excretion when administered to a human being, and are therefore useful in therapy in the treatment of diseases in which ion, e.g. iron, aluminum, levels in the body have elevated or toxic levels. These diseases for iron overload include, for example, thalassemia major, sideroachrestic anemia, Blackfan-Diamond anemia, aplastic anemia, sickle cell anemia, hemolytic anemias and hemosiderosis brought about by multiple blood transfusions or such condition when brought about by treatment of an anemia found in kidney-damaged patients undergoing renal dialysis.

Another aspect of the present invention relates to compounds of formula I as is described herein which liberate deferoxamine in the body to generally chelate any trivalent metal, such as iron, aluminum, chromium, gallium, ytterbium and indium, for subsequent excretion, which is useful in the treatment of conditions (which is equvalent to diseases) in which the elevated levels of metal ion in the body cause or exacerbate disease conditions. The compounds of formula I are useful as oral pharmaceuticals in the treatment of Alzheimer's and related diseases in which elevated aluminum levels have been found in the body, particularly the brain. Diseases or conditions having elevated aluminum body levels also include senile dementia and dialysis encephalopathy.

Thus other aspects of the invention concern pharmaceutical preparations incorporating the compounds of formula I, dosage forms thereof and methods of treatment of the aforementioned conditions employing these preparations and/or dosage forms.

Another aspect of this invention is a process for the preparation of the compounds of formula I, as is described in greater detail hereinafter.

## Definitions

"Acyl" is defined to refer to a group having the structure —C(=O)—$R_5$ wherein $R_5$ is selected from $C_{1-25}$ alkyls and substituted $C_1$—$C_{25}$ alkyls having 1 to 3 protons replaced by alkoxyl or halogen.

"Acylating agent" refers to a compound containing the group —C(=O)—$R_5$ which can react and insert an "acyl" into deferoxamine. Representative agents include, for example, acyl halides, acyl anhydrides, mixed acyl anhydrides and mixtures thereof. When different acylating agents are employed herein they may include alkyl acyl of the same category (e.g. acetyl chloride and propionyl chloride) or alkyl acyl substituted at one to three positions, and alkyl acyl, e.g., propionyl chloride and 2-chloropropionyl chloride.

"Alkyl" refers to a branched or unbranched saturated hydrocarbon containing 1 to 25 carbon atoms, such as, for example, methyl, ethyl, n-propyl, i-propyl, n-butyl, i-butyl, s-butyl, t-butyl, n-heptyl, i-heptyl, n-octyl, nonyl, decyl, undecyl, tridecyl, pentadecyl, heptadecyl and pentacosanyl.

"Substituted alkyl" refers to an "alkyl" group, wherein at positions on the linear or branched structure one to three protons have been replaced by alkoxyl or halogen.

"Halo" or "halogen" refers to fluoro, chloro, bromo or iodo.

The compounds of this present invention are generally named according to the IUPAC or *Chemical Abstracts* nomenclature. Thus, deferoxamine may be named N' - [5 - [[4 -[[ -5 - (acetylhydroxamino)pentyl]amino]1,4 - dioxobutyl]hydroxyamino]pentyl] - N - (5 - aminopentyl) - N - hydroxybutanediamide; or N - [5 - [3[(5-aminopentyl)hydroxylcarbamoyl]propionamido]pentyl] - 3 -

[[5 - (N - hydroxyacetamido)pentyl] - carbamoyl]propionohydroxamic acid; or 1 - amino - 6,17 - dihydroxy - 7,10,18,21 - tetraoxo - 27 - (N - acetylhydroxylamino)-6,11,17,22 - tetraazaheptaeicosane.

Because of the obvious complexity of the names for the substituted structures of deferoxamine, a shorthand form based upon the last written name above is used for the present invention. Therefore, the 1-amino group, when substituted by acyl ($R_1$—), is designated as N-acyl (—N—$R_1$). The hydroxamic acid hydrogen at the 6-position, when substituted by acyl ($R_2$—) is designated as O-acyl (—O—$R_2$). The hydroxamic acid hydrogen at the 17-position, when substituted by acyl ($R_3$—) is designated as O-acyl (—O—$R_3$). And the hydroxamic acid hydrogen of the "27-(N-acetylhydroxylamino)" when substituted by acyl ($R_4$—) is designated as (—O—$R_4$). The second hydrogen on the 1-amino group and the hydrogens on the amide nitrogens at the 11 and 22 positions, when substituted by acyl, are also N-acyls designated by $R_6$(—N—$R_6$), $R_7$(—N—$R_7$) and $R_8$(—N—$R_8$), respectively.

Thus in formula I, when $R_1$ is acetyl, and $R_2$, $R_3$ and $R_4$ are each n-octanoyl, the compound name is N-acetyl-O,O,O-trioctanoyldeferoxamine. When $R_1$ is isovaleryl, $R_2$ is acetyl (i.e., $R_5$ here is —$CH_3$), $R_3$ is butyryl ($R_5$ here is —$CH_2CH_2CH_3$) and $R_4$ is n-octanoyl [$R_5$ here is —$CH_2(CH_2)_5CH_3$], the compound name is N-isovaleryl-O,O,O-acetylbutyryl-n-octanoyldeferoxamine. If the amino group or any combination of the hydroxamic acid groups are unsubstituted, the unsubstituted position is designated as N-hydrogen (N—H) or —O-hydrogen (—O—H), respectively, reading $R_2$, $R_3$, and $R_4$, from left to right for the compound of formula I. In the compound when $R_1$ is acetyl, $R_2$, $R_3$, $R_4$, and $R_6$ are each octenyl and $R_7$ and $R_8$ are H, the compound is named H-acetyl-O,O,O,N,H,H-tetraoctanoyl-deferoxamine.

Although not understood with certainty, it appears that the best results are obtained when the total number of carbon atoms in the groups $R_1$, $R_2$, $R_3$, $R_6$, $R_7$ and $R_8$ of formula I is between 10 and 60, preferably between 12 and 40, and especially between 14 and 30. Structures of formula I where the total of the carbon atoms in the groups $R_1$, $R_2$, $R_3$, $R_4$, $R_6$, $R_7$ and $R_8$ are less than 9 have not yet achieved good results, perhaps because deferoxamine derivatives having these smaller acyls are not sufficiently absorbed through the membranes of the digestive tract. Structures of formula I wherein the total of the carbon atoms of $R_1$, $R_2$, $R_3$, $R_4$, $R_6$, $R_7$ and $R_8$ is greater than 63 have not yet achieved good results, perhaps because the molecules are not sufficiently soluble in the fluids within the digestive tract to be absorbed into the body as the prodrug to be cleaved to produce deferoxamine.

The compounds of formula I, prepared according to the procedures described herein and which achieve good results in reducing the amount of tissue iron or aluminum in a human being, are found in Table I.

TABLE I

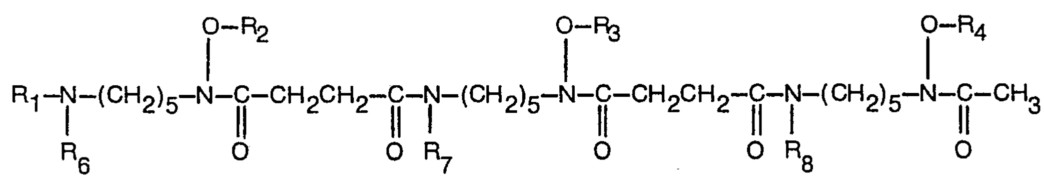

DEFEROXAMINE DERIVATIVES

| Compound Group | $R_1$ | $R_2$ | $R_3$ | $R_4$ | $R_6$ | $R_7$ | $R_8$ |
|---|---|---|---|---|---|---|---|
| | Number of Carbon Atoms | | | | | | |
| 1 | 2 | 3 | 3 | 3 | 0 | 0 | 0 |
| 1A | 2 | 2 | 2 | 2 | 2 | 0 | 0 |
| 1B | 2 | 2 | 2 | 2 | 2 | 0 | 2 |
| 1C | 2 | 2 | 2 | 2 | 2 | 2 | 2 |
| 2 | 2 | 4 | 4 | 4 | 0 | 0 | 0 |
| 3 | 2 | 5 | 5 | 5 | 0 | 0 | 0 |
| 4 | 2 | 6 | 6 | 6 | 0 | 0 | 0 |
| 5 | 2 | 7 | 7 | 7 | 0 | 0 | 0 |
| 6 | 2 | 8 | 8 | 8 | 0 | 0 | 0 |

TABLE I

DEFEROXAMINE DERIVATIVES

| Compound Group | Number of Carbon Atoms R$_1$ | R$_2$ | R$_3$ | R$_4$ | R$_6$ | R$_7$ | R$_8$ |
|---|---|---|---|---|---|---|---|
| 6A | 2 | 8 | 8 | 8 | 8 | 0 | 0 |
| 6B | 2 | 8 | 8 | 8 | 0 | 0 | 8 |
| 6C | 2 | 8 | 8 | 8 | 8 | 0 | 8 |
| 6D | 2 | 8 | 8 | 8 | 8 | 8 | 8 |
| 8 | 3 | 4 | 4 | 4 | 0 | 0 | 0 |
| 9 | 3 | 5 | 5 | 5 | 0 | 0 | 0 |
| 10 | 3 | 6 | 6 | 6 | 0 | 0 | 0 |
| 11 | 3 | 8 | 8 | 8 | 0 | 0 | 0 |
| 12 | 4 | 3 | 3 | 3 | 0 | 0 | 0 |
| 14 | 4 | 5 | 5 | 5 | 0 | 0 | 0 |
| 15 | 4 | 6 | 6 | 6 | 0 | 0 | 0 |
| 16 | 4 | 8 | 8 | 8 | 0 | 0 | 0 |
| 17 | 5 | 3 | 3 | 3 | 0 | 0 | 0 |
| 18 | 5 | 4 | 4 | 4 | 0 | 0 | 0 |
| 20 | 5 | 6 | 6 | 6 | 0 | 0 | 0 |
| 21 | 5 | 8 | 8 | 8 | 0 | 0 | 0 |
| 22 | 6 | 3 | 3 | 3 | 0 | 0 | 0 |
| 23 | 6 | 4 | 4 | 4 | 0 | 0 | 0 |
| 24 | 6 | 5 | 5 | 5 | 0 | 0 | 0 |
| 25A | 6 | 5 | 5 | 5 | 5 | 0 | 0 |
| 27 | 6 | 8 | 8 | 8 | 0 | 0 | 0 |
| 28 | 7 | 3 | 3 | 3 | 0 | 0 | 0 |
| 29 | 7 | 4 | 4 | 4 | 0 | 0 | 0 |
| 30 | 7 | 5 | 5 | 5 | 0 | 0 | 0 |
| 31 | 7 | 6 | 6 | 6 | 0 | 0 | 0 |
| 33 | 8 | 3 | 3 | 3 | 0 | 0 | 0 |
| 34 | 8 | 4 | 4 | 4 | 0 | 0 | 0 |
| 35 | 8 | 5 | 5 | 5 | 0 | 0 | 0 |
| 36 | 8 | 6 | 6 | 6 | 0 | 0 | 0 |

TABLE I

DEFEROXAMINE DERIVATIVES

| Compound Group | Number of Carbon Atoms | | | | | | |
|---|---|---|---|---|---|---|---|
| | $R_1$ | $R_2$ | $R_3$ | $R_4$ | $R_6$ | $R_7$ | $R_8$ |
| 37A | 8 | 8 | 8 | 8 | 8 | 0 | 0 |
| 37B | 8 | 8 | 8 | 8 | 8 | 0 | 8 |
| 37C | 8 | 8 | 8 | 8 | 8 | 8 | 8 |

Preferred compounds of formula I found in the "Compound Groups" in Table I are those compounds wherein in $R_1$, $R_5$ is $C_{1-25}$ alkyl, and $R_2$, $R_3$, $R_4$, $R_6$, $R_7$ and $R_8$ are independently selected from hydrogen or acyl of the formula —(C=O)—$R_5$, where $R_5$ is independently selected for each from $C_{1-25}$ alkyl groups. More preferred are the $C_{1-7}$ alkyl groups. Especially preferred are those compounds where $R_6$, $R_7$ and $R_8$ are hydrogen [such that at least one of $R_2$, $R_3$ and $R_4$ is an acyl of the formula —(C=O)—$R_5$ different from $R_1$]. Preferred groups include Compound Groups 1A, 37A, 37B and 37C wherein $R_1$, $R_2$, $R_3$, $R_4$, $R_6$, $R_7$ and $R_8$ each contain the same number of carbon atoms or represent hydrogen. More preferred are those groups wherein $R_5$ is $C_{1-25}$ alkyl, particularly $C_{1-7}$ alkyl and especially where $R_5$ is the same alkyl group. These compounds where $R_5$ is alkyl are preferred to be orally administered to treat the iron and aluminum related diseases described herein.

Preferred compounds of formula I also include those wherein up to five of $R_2$, $R_3$, $R_4$, $R_6$, $R_7$ and $R_8$ are hydrogen, more preferably the hydrogens are found on positions $R_6$, $R_7$ and $R_8$. See, for example, Table II.

TABLE II

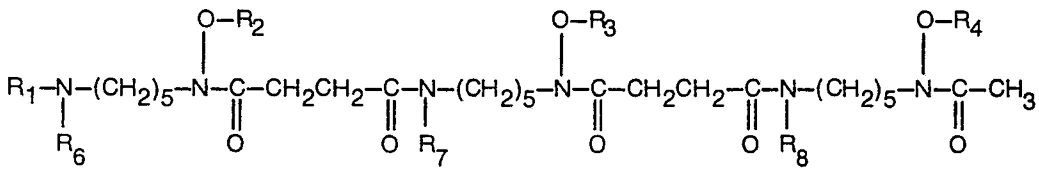

DEFEROXAMINE DERIVATIVES

| Compound Group | Number of Carbon Atoms[a] | | | | | | |
|---|---|---|---|---|---|---|---|
| | $R_1$ | $R_2$ | $R_3$ | $R_4$ | $R_6$ | $R_7$ | $R_8$ |
| 1 | 2 | 8 | 8 | 0 | 0 | 0 | 0 |
| 1A | 2 | 8 | 8 | 0 | 8 | 0 | 0 |
| 1B | 2 | 8 | 8 | 0 | 8 | 0 | 8 |
| 2 | 2 | 8 | 0 | 8 | 0 | 0 | 0 |
| 3 | 2 | 0 | 8 | 8 | 0 | 0 | 0 |
| 4 | 2 | 0 | 0 | 8 | 0 | 0 | 0 |
| 5 | 2 | 0 | 8 | 0 | 0 | 0 | 0 |
| 6 | 2 | 8 | 0 | 0 | 0 | 0 | 0 |
| 7 | 4 | 4 | 4 | 0 | 0 | 0 | 0 |
| 8 | 4 | 6 | 0 | 6 | 0 | 0 | 0 |

TABLE II

DEFEROXAMINE DERIVATIVES

| Compound Group | Number of Carbon Atoms[a] R₁ | R₂ | R₃ | R₄ | R₆ | R₇ | R₈ |
|---|---|---|---|---|---|---|---|
| 9 | 4 | 0 | 8 | 8 | 0 | 0 | 0 |
| 10 | 5 | 5 | 5 | 0 | 0 | 0 | 0 |
| 11 | 5 | 0 | 6 | 6 | 0 | 0 | 0 |
| 12 | 6 | 6 | 6 | 0 | 0 | 0 | 0 |
| 13 | 6 | 5 | 5 | 0 | 0 | 0 | 0 |
| 14 | 6 | 8 | 8 | 0 | 0 | 0 | 0 |
| 15 | 8 | 8 | 8 | 0 | 0 | 0 | 0 |
| 16 | 8 | 8 | 0 | 8 | 0 | 0 | 0 |
| 17 | 8 | 0 | 8 | 8 | 0 | 0 | 0 |
| 17A | 8 | 8 | 0 | 8 | 8 | 8 | 0 |
| 17B | 8 | 8 | 8 | 0 | 8 | 0 | 8 |
| 17C | 8 | 8 | 0 | 8 | 8 | 0 | 0 |
| 18 | 8 | 4 | 4 | 0 | 0 | 0 | 0 |
| 19 | 8 | 5 | 5 | 0 | 0 | 0 | 0 |
| 20 | 8 | 6 | 6 | 0 | 0 | 0 | 0 |
| 20A | 8 | 6 | 6 | 0 | 6 | 0 | 0 |
| 20B | 8 | 6 | 0 | 6 | 6 | 0 | 6 |
| 21 | 6 | 4 | 0 | 4 | 0 | 0 | 0 |
| 22 | 5 | 4 | 0 | 4 | 0 | 0 | 0 |
| 23 | 4 | 5 | 0 | 5 | 0 | 0 | 0 |
| 24 | 3 | 3 | 3 | 0 | 0 | 0 | 0 |
| 25 | 3 | 4 | 4 | 0 | 0 | 0 | 0 |
| 26 | 3 | 5 | 0 | 5 | 0 | 0 | 0 |
| 27 | 3 | 4 | 0 | 4 | 0 | 0 | 0 |
| 28 | 2 | 4 | 0 | 4 | 0 | 0 | 0 |
| 29 | 2 | 5 | 5 | 0 | 0 | 0 | 0 |
| 30 | 2 | 6 | 0 | 6 | 0 | 0 | 0 |

a. When the carbon atom number is 0, the group $R_2$, $R_3$, $R_4$, $R_6$, $R_7$ and $R_8$ contains 0 carbon atoms, and is a hydrogen (—H).

Because of the present difficulty of separating some of the isomers of the products described in Table I and Table II, this invention includes mixtures of compounds which would normally be expected in the reaction products described in the examples below. For instance, if an excess of acylating agent is used as shown in Table I, then a mixture of compounds such as 6, 6A, 6B, 6C and 6D may be present. If in the Compound Group in Table II numbers 15, 16, 17, 17A, 17B and 17C are present as reaction products of the acylation of deferoxamine using a limited amount of $R_1$=8 [(i.e., $R_5$=7 carbon atoms) acylating agent], then the mixture of isomers may be used in therapy. These mixtures of isomers may be separated by, e.g. high pressure liquid chromatography or may be used in a pharmaceutical composition or method of treatment as a mixture of 2 or more isomers.

Presently preferred embodiments of the present invention as an oral pharmaceutical composition and method of treatment, include those compounds of formula I wherein $R_2$, $R_3$ and $R_4$ are identical $C_1$—$C_{25}$ alkyl acyl groups, especially $C_{1-7}$ alkyl acyls. Especially preferred are those compounds where acyl is —C(=O)—$R_5$, and $R_5$ is n-propyl, n-butyl, t-butyl, n-pentyl, n-hexyl or n-heptyl, such that at least one of $R_2$, $R_3$ and $R_4$ is an acyl of the formula —C(=O)—$R_5$ different from $R_1$, when $R_6$, $R_7$ and $R_8$ are each hydrogen.

A preferred compound is where $R_1$ is an acetyl, $R_2$, $R_3$ and $R_4$ are each acyl where $R_5$ is n-heptyl; and two of $R_6$, $R_7$ and $R_8$ are hydrogen and the other is acyl where $R_5$ is n-heptyl.

A particularly preferred embodiment is the compound where $R_1$ is —C(=O)—$R_5$ and $R_5$ is undecyl, and $R_2$, $R_3$, and $R_4$ are each —C(=O)—$R_5$ wherein $R_5$ is propyl.

Preferred compounds of the embodiments of formula I described above for a pharmaceutical composition and for a method of treatment of ion, e.g. iron or aluminum, overload diseases as those where R6, R7 and R8 are each hydrogen, such that at least one of $R_2$, $R_3$ and $R_4$ is an acyl of the formula —C(=O)—$R_5$ different from $R_1$.

An additional embodiment of the present invention describes a pharmaceutical composition useful for treating one or more diseases or conditions in a human being, related to excess iron in the blood and/or tissue, which comprises using a therapeutically effective amount of a compound of formula I in admixture with a pharmaceutically acceptable excipient.

Additional preferred embodiments include the pharmaceutical compositions including the compound of formula I wherein $R_1$ is one acyl group of the formula —C(=O)—$R_5$ containing 2—8 carbon atoms and $R_2$=$R_3$=$R_4$ and $R_6$=$R_7$=$R_8$ are all a different acyl group of the formula —C(=O)—$R_5$ wherein $R_5$ contains 1 to 7 carbon atoms. Preferred compounds include those where $R_1$ is acetyl and $R_2$=$R_3$=$R_4$ where $R_5$ is ethyl, n-propyl, n-butyl, i-butyl, t-butyl, n-pentyl, n-hexyl, or n-heptyl and $R_6$, $R_7$ and $R_8$ are hydrogen. Especially preferred compounds are N-acetyl-O,O,O-tri-n-octanoyldeferoxamine and N-acetyl-O,O,O,N,H,H-tetra-n-octanoyldeferoxamine.

Still another embodiment of the present invention describes a process for the preparation of the compounds of formula I which process comprises contacting the unsubstituted deferoxamine wherein $R_1$, $R_2$, $R_3$, $R_4$, $R_6$, $R_7$ and $R_8$ are each hydrogen with a suitable acylating agent in the presence of a strong base having a pK of 9 to 11, treating the product with a weak base to form the N-acyl-O,O,O-trihydrogendeferoxamine; and treating this material with an excess of one or more different acylating agents to form the compound of formula I. In a preferred embodiment the first suitable acylating agent is $R_5$—C(=O)—X or $R_5$—(C=O)OC(C=O)—$R_5$ where $R_5$ contains 1 to 7 carbon atoms and X is a halogen; the weak base has a $pK_b$ value of 4 to 6; and the second different acylating agent is $R_5$—C(=O)—X or $R_5$(C=O)OC(C=O)$R_5$ wherein $R_5$ contains 1 to 7 carbon atoms and X is halogen. A particularly preferred embodiment is the process wherein $R_5$ of the first acylating agent contains one carbon atom; the weak base is ammonia; and in the different acylating agent $R_5$ contains 4 to 7 carbon atoms. An especially preferred embodiment is the process wherein the first acylating agent is acetyl chloride or acetic anhydride; the base is anhydrous ammonia; and the different acylating agent is octanoyl chloride. The reaction products of formula I may be separated using HPLC or equivalent means.

Process for Preparation

In Reaction Sequence 1, deferoxamine (Ia), as described by M. Windholz, *Ed.* in *The Merck Index,* published by Merck Co., Inc. of Rahway, New Jersey in 1976 (p. 374), is used as a starting material.

REACTION SEQUENCE 1

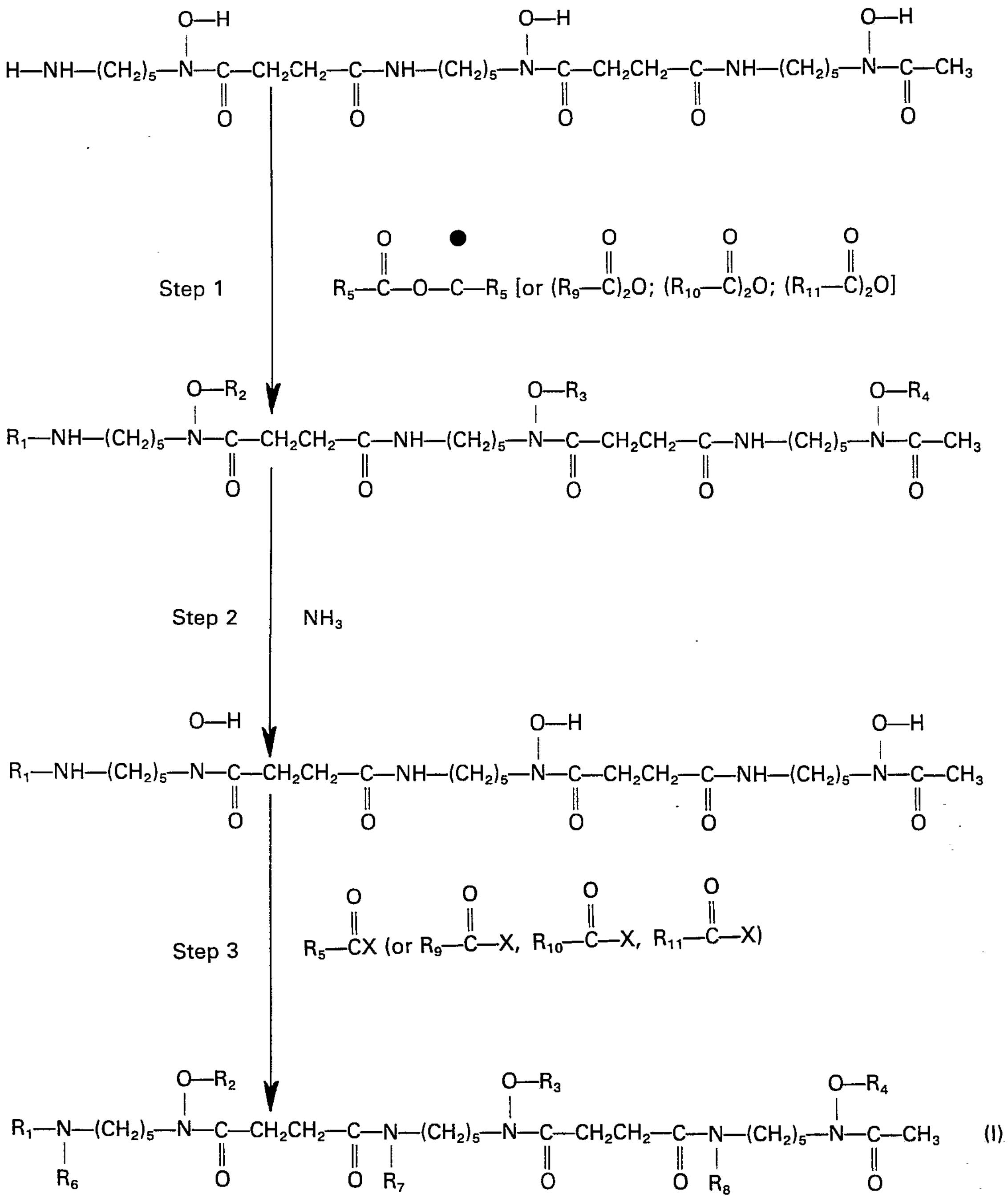


(Ia)

(Ib)

(Ic)

(I)

In Step 1, deferoxamine (Ia) is treated with an excess of acyl anhydride, $[R_9(C{=}O)]_2O$, $[R_{10}(C{=}O)]_2O$ and $[R_{11}(C{=}O)]_2O$, in the presence of the alkaline salt of the anhydride in an alcoholic solvent. After about 12 to 24 hours, the solvent and acyl acid are removed under reduced pressure to yield Ib. $R_9$, $R_{10}$ and $R_{11}$ independently may be the same or different groups as is described herein for $R_5$. Thus anhydrides $R_9{-}C({=}O){-}X$, $R_{10}{-}C({=}O){-}X$ and $R_{11}{-}C({=}O){-}X$ as acyl halides may replace the corresponding anhydride and may be used alone or as a mixture to acylate deferoxamine. If these procedures are used, then it is possible to convert compound of formula Ia to the compound of formula I in one step. [See Example 2(1) (m) and 2(3) (m) below.] The reaction product is a mixture which may be used, as a mixture, as a pharmaceutical agent, as is described herein. On the other hand, the products may be separated by methods described herein below and used separately.

Alternatively, deferoxamine (Ia) may be tetra- up to and including hepta-acylated using an acyl halide. Deferoxamine is suspended in a solution of water/solvent (i.e., water/dioxane, about 50/50) and the pH is adjusted to about 9 using a strongly basic solution, preferably 4 to 7N sodium hydroxide. In small portions, the acyl halide in a solvent, such as dioxane, is added dropwise keeping the pH at about 9. Water and a

chlorinated solvent, such as chloroform, may be needed to keep the reactants in solution. Strong agitation of the reaction mixture is necessary. The dioxane (and chloroform) phase is removed, washed, dried and removed *in vacuo* to produce a mixture of compounds of formula Ib and (I). Step 1 and Step 3 below, using acyl halide are often referred to as Schotten-Baumann reaction, which is described in the art.

In Step 2, the compound of formula Ic is obtained by dissolving the N-acyl-O,O,O-triacyl (to hepta-acyl) product of formula Ib in an excess of an ethereal alcoholic solvent, such as methanol, and cooling to −20°C to +20°C, preferably 0°C, the reaction mixture is subsequently saturated with a base, preferably gaseous ammonia. After maintaining the reaction mixture at −20°C to +20°C, preferably at ambient temperature, and allowed to stir for 24 to 48 hours, the solvent is decanted and the product, usually as a solid, is recovered, washed twice with boiling hexane, and the resulting solid is and dried under reduced pressure. After recrystallization from alcohol/water solution, the product is recovered and air dried.

In Step 3, compound Ic is suspended in a solution of water/solvent (i.e., water/chloroform 50/50). The solution is adjusted to about pH of 9 using strong base, preferably 3—7N sodium hydroxide solution. To this mixture is added dropwise a solution of the acyl halide, preferably the chloride, in a solvent such as chloroform. The pH of the solution is continuously monitored and is maintained at pH of 9. The layer of chlorinated solvent is removed, washed, dried, filtered, and evaporated *in vacuo* to produce an oily or waxy product, the compound of formula I.

Isolation and purification of the compounds and intermediates described herein can be effected, if desired, by any suitable separation or purification procedure such as, for example, filtration, extraction, crystallization, column chromatography, high pressure liquid chromatography (HPLC), thin-layer chromatography or thick-layer chromatography, dry column chromatography or a combination of these procedures. Specific illustrations of suitable separation and isolation techniques can be had by reference to the examples herein below. Other equivalent separation or isolation procedures, however, could also be used.

In the preparation of the mixture of compounds of formula I, separation, purification, and identification of the fully acylated or 49 possible partially acylated derivatives of the deferoxamine is difficult, uneconomic and sometimes impossible with present separation techniques. Therefore, this invention includes mixtures of compounds of formula I wherein the groups, $R_1$, $R_2$, $R_3$, $R_4$, $R_6$, $R_7$ and $R_8$ are hydrogen or acyl as is defined and as limited above. The mixture of isomers is administered as part of a pharmaceutical composition to a person in the same manner that an essentially pure compound of formula I would be administered.

Although not known with certainty, it appears that of the N-acylated groups $R_6$, $R_7$, and $R_8$, N—$R_6$ is formed first because it is the least sterically hindered of the three available amides. However, it is to be understood that with present analytical techniques, it is not known with certainty which isomer(s) of the derivatives N—$R_6$, N—$R_7$ and N—$R_8$ is present.

The acyl halides and anhydrides, solvents, reagents and the like described herein are available according to *Chemical Sources*, published by Directories Publishing Company, Inc., Flemington, New Jersey in 1979. Those halides or anhydrides not available are prepared according to methods known or adapted from the art, see for example, R. Morrison and R. Boyd, *Organic Chemistry*, 3rd ed., published by the Benjamin Co. in 1976.

Utility and Administration

Administration of the compound of this invention can be via any of the accepted modes of administration for therapeutic agents. These methods include oral, parenteral, transdermal, subcutaneous and other systemic modes. The preferred method of administration is oral.

Depending on the intended mode, the composition may be in many forms, for example, solid, semisolid, or liquid dosage forms, including tablets, time release agents, pills, capsules, suspensions and solutions. The compositions will include a conventional pharmaceutical excipient and an active compound of formula I or the pharmaceutically acceptable salts thereof and may, in addition, include other medicinal agents, pharmaceutical agents, carriers, adjuvants and diluents.

The amount of the active compound of formula I administered will, of course, be dependent on the molecular weight of selected compound, the subject being treated, the subject's weight, the severity of the affliction, the manner of the administration and the judgment of the prescribing physician. However, an effective dose is in the range of 25—200 mg/kg/day, preferably 125 mg/kg/day. For an average 70 kg human, those dosages would amount to 1.5 to 14 g/day, or preferably 9 g/day.

For solid compositions, conventional non-toxic solids include for example, pharmaceutical grades of mannitol, lactose, starch, magnesium stearate and cellulose may be used. Liquid pharmaceutically administratable compositions can be prepared by dissolving or dispersing a compound of formula I and optional pharmaceutical adjuvants in an excipient, such as water, glycerol, ethanol and vegetable oil to form a suspension.

Actual methods of preparing such dosage forms are known, or will be apparent to those skilled in the art; see, for example, *Remington's Pharmaceutical Sciences*, Mack Publishing Company, Easton, Pennsylvania, 15th Edition, 1975.

The following preparations and examples serve to illustrate the invention.

Example 1

(1) Preparation of N-Acetyl O,O,O-triacetyldeferoxamine

(a) Deferoxamine mesylate (13.1 g) and 1.66 g of anhydrous sodium acetate are dissolved in 200 ml of methanol. The methanol solution is boiled to complete the solution. The reaction mixture is then rapidly cooled to ambient temperature and treated immediately (before crystallization starts) with 180 ml of acetic anhydride. The mixture is maintained overnight (about 16 hrs) in the absence of moisture and then concentrated under vacuum to produce an oily residue. This residue is freed from acetic acid by evaporation under vacuum using two portions of 200 ml of butanol. The oily residue is air dried for several days to produce 14.1 g of crude N-acetyl O,O,O-triacetyldeferoxamine. The proton magnetic resonance spectrum of the recrystallized product is consistent with this structure; [structural unit, parts per million (ppm) downfield from tetramethylsilane (TSM) reference]:

(for C—H absorption):
—N—C—C—$CH_2$—C—C—N—O—: 1.44;
—N—C—C—C—$CH_2$—C—N—O: 1.52;
—N—C—$CH_2$—C—C—C—N—O: 1.58;
$CH_3$—C(=O)—N—(OCO—C—): 1.95;
$CH_3$—C(=O)—NH—C—C—C—: 1.99;
—C—C(=O)—N—(O—CO—$CH_3$): 2.17;
—N—C(=O)—$CH_2$—$CH_2$—(C=O)—N—: 2.56;
—C—C—$CH_2$—N(—O)—(C=O)—: 3.22;
—C—(C=O)—NH—$CH_2$—C—: 3.70; and
(for the N—H absorption):
—C—C(=O)—NH—C—C—: 6.28.

(b) Similarly proceeding as in Subpart (a) above but substituting a stoichiometrically equivalent amount of

propionyl anhydride;
butyryl anhydride;
valeryl anhydride;
isovaleryl anhydride;
octanoyl anhydride;
dodecanoyl anhydride;
palmitoyl anhydride;
stearoyl anhydride; or
hexacosanoyl anhydride;

instead of acetic anhydride, there is obtained the corresponding

N-propionyl-O,O,O-tripropionyldeferoxamine;
N-butyryl-O,O,O-tributyryldeferoxamine;
N-valeryl-O,O,O-trivaleryldeferoxamine;
N-isovaleryl-O,O,O-trivaleryldeferoxamine;
N-octanoyl-O,O,O-trioctanoyldeferoxamine;
N-dodecanyl-O,O,O-tridodecanoyldeferoxamine;
N-palmitoyl-O,O,O-tripalmitoyldeferoxamine;
N-stearyl-O,O,O-tristearoyldeferoxamine; or
N-hexacosanoyl-O,O,O-tri(hexacosanoyl)deferoxamine.

It is often necessary to employ larger volumes of solvents to keep the substituted deferoxamine in solution and to obtain more complete acylation of the three hydroxamic acids.

(c) Similarly, when proceeding as in Subpart (a) above but substituting less than a stoichiometrically equivalent amount of the acyl anhydride, there is obtained a compound wherein the N-acyl group is formed and a mixture containing less than complete acylation of the hydroxamic acid groups of deferoxamine. Such mixtures when acetic anhydride is used include the following compounds:

N-acetyl-O,O,O-hydrogendiacetyldeferoxamine
and
N-acetyl-O,O,O-dihydrogenacetyldeferoxamine.

The exact positions of the acetyl and hydrogen groups on the hydroxamines is not yet established.

(d) Similarly proceeding as in Subpart (a) above but substituting a stoichiometrically equivalent amount of a mixture of the following anhydrides:

acetic anhydride, propionic anhydride, and butyric anhydride;

instead of acetic anhydride, there is obtained a mixture of corresponding tetraacetyl derivatives including:

N-acetyl-O,O,O-acetylpropionylbutyryldeferoxamine;
N-butyryl-O,O,O-acetylpropionylbutyryldeferoxamine; and
N-propionyl-O,O,O-butyrylpropionylacetyldeferoxamine.

The exact positions of the acyl groups in the hydroxamines is not yet established with certainty.

(2) Preparation of N-Acetyl-O,O,O-trihydrogendeferoxamine

(a) N-Acetyl-O,O,O-triacetyldeferoxamine (prepared in Example 1(1)) (10.0 g) is taken up in 200 ml of methanol and 500 ml of ether, cooled to 0°C, and the solution is saturated with anhydrous gaseous

ammonia. The reaction mixture is kept at 0°C for 6 hr, and then at ambient temperature overnight (about 16 hr). The ammonia-containing methanol/ether is decanted and the resulting colorless crystalline solid is washed twice with boiling hexane and is dried in vacuum, crude yield 8.1 g. After two recrystallizations using methanol/water (60/40), there are obtained about 7.0 grams of N-acetyl-O,O,O-trihydrogendeferoxamine, m.p. 179—181°. The infrared spectrum and proton magnetic resonance spectrum are consistent with the structure.

(b) Similarly, proceeding as in Subpart (a) of this Example, but substituting a stoichiometrically equivalent amount of:

N-propionyl-O,O,O-tripropionyldeferoxamine;
N-butyryl-O,O,O-tributyryldeferoxamine;
N-valeryl-O,O,O-trivaleryldeferoxamine;
N-octanoyl-O,O,O-trioctanoyldeferoxamine;
N-palmitoyl-O,O,O-tripalmitoyldeferoxamine;

or

N-pentacosanyl-O,O,O-tripentacosanoyldeferoxamine instead of the N-acetyl-O,O,O-triacetyl-deferoxamine, there is obtained the corresponding

N-propionyl-O,O,O-trihydrogendeferoxamine;
N-butyryl-O,O,O-trihydrogendeferoxamine;
N-valeryl-O,O,O-trihydrogendeferoxamine;
N-octanoyl-O,O,O-trihydrogendeferoxamine;
N-palmitoyl-O,O,O-trihydrogendeferoxamine;

or

N-pentacosanoyl-O,O,O-trihydrogendeferoxamine.

(3) Preparation of N-Acetyl-O,O,O-tripalmitoyldeferoxamine

(a) N-Acetyl-O,O,O-trihydrogendeferoxamine (from Example 1(2)) (6.0 g) is suspended in a solution of 40 ml of water and 50 ml of dioxane. The well-agitated suspension is adjusted to pH of 9 using 5N sodium hydroxide solution. To this mixture is added in 10 ml portions, a solution of 16.5 g of palmitoyl chloride in 60 ml of dioxane. The pH of the mixture is maintained by the addition of a 5N sodium hydroxide solution after each 10 ml portion of the acyl chloride solution. After 40 ml of the palmitoyl chloride solution are added, 50 ml of water and 200 ml of chloroform are added to facilitate the mixing of the solution. After the addition of the palmitoyl chloride solution is completed, the reaction mixture is stirred for 1 hr, with periodic monitoring to maintain a pH of 9. The reaction mixture is then diluted with 150 ml of water and 500 ml of chloroform, and centrifuged to separate the phases. The white material present at the liquid interface is discarded. [The aqueous phase is separated and extracted twice with 250 ml of chloroform. Essentially no product is obtained upon removal of the chloroform]. The chloroform phase contained a white solid which is removed using additional centrifugation. The combined chloroform layers are washed twice with saturated sodium bicarbonate solution, twice with saturated sodium chloride solution, dried over anhydrous sodium sulfate, filtered and evaporated using reduced pressure. About 14 g of a crude, creamy white waxy solid is obtained which is highly soluble in chloroform. The waxy solid was triturated twice with 100 ml of ether to remove the palmitic acid formed. The insoluble residue, 11.8 g, was recrystallized from methanol/ethanol (3/1). The solid is air dried to produce 6.1 g of solid N-acetyl-O,O,O-tripalmitoyldeferoxamine. The nuclear magnetic resonance spectrum is consistent with the structure.

(3A) Preparation of N-Acetyl-O,O,O,N,H,H-tetraoctanoyldeferoxamine

(a) N-Acetyl-O,O,O-trihydrogendeferoxamine (from Example (1)2) (3.0 g) is suspended in a mixture of 100 ml of water and 150 ml of chloroform. The suspension is adjusted to pH 9 using 5N sodium hydroxide. To the well-agitated mixture is added dropwise, over a period of 45 min., a solution of 7.3 g octanoyl choride in 50 ml of chloroform. The mixture is continuously maintained at pH 9 by the addition of 5N sodium hydroxide as necessary. After the addition of the octanoyl chloride is completed, the reaction mixture is stirred for 1 hr, with periodic monitoring to maintain a pH of 9. The chloroform phase is removed and the aqueous phase is extracted two times with 100 ml chloroform, centrifuging to break the emulsion when necessary. The combined chloroform layers are washed twice with saturated sodium bicarbonate solution, twice with saturated sodium chloride solution, dried over anhydrous sodium sulfate, filtered and evaporated under reduced pressure. The syrupy residue is triturated twice with 50 ml of ether to remove the octanoic acid formed. The insoluble residue (2.6 g) is dissolved in dichloromethane and fractionated by HPLC.

Four main fractions are obtained at the following retention times: 2.4 min (11% relative abundance), 3.4 min (29%), 4.7 min (15%) and 7.0 min (40%) using a 5 micron silica-CN column and a methanol gradient from 2% to 5% over 12 min in a mixture containing 25% chlorobutane and iso-octane at a flow rate of 2 ml/min.

Fraction number 3 (4.7 min) (amounting to 313 mg) was determined by 300 MHz NMR to be N-acetyl-O,O,O-tetraoctanoyl-N-octanoyldeferoxamine. Fraction number 4 (7.0 min) (amounting to 799 mg) was determined by 300 MHz NMR to be N-acetyl-O,O,O-trioctanoyldeferoxamine. The 300 MHz proton nuclear magnetic resonance (NMR) spectral results are shown below in Table III.

TABLE III

Proton NMR Results

| Chemical Shift | Assignment | Fract. 3 | | Fract. 4 | |
|---|---|---|---|---|---|
| | | Actual | Theory | Actual | Theory |
| 0.9 | $CH_3(CH_2)_5$— | 12. | 12. | 9. | 9. |
| 1.4 | $CH_3(CH_2)_5CH_2$— | 62. | 58. | 54. | 48. |
| 1.8 | —$NCH_2(CH_2)_3CH_2N$— | | | | |
| 2.0 | $CH_3$—(C=O)—N— | 5.5 | 6. | 6.2 | 6. |
| 2.1 | —O—N—(C=O)—$CH_3$ | | | | |
| 2.7 | $(O{=}C)CH_2CH_2(C{=}O)$ N—O—(C=O)—$CH_2$— | 15.5 | 16. | 15.4 | 14. |
| 3.3 | O—N—$CH_2$—C | 4.6 | 6. | 6.9 | 6. |
| 3.7 | (O=C)—N—$CH_2$—C | 9. | 6. | 7.7 | 6. |
| | (proton total) | 108.6 | 106. | 99.2 | 92. |

a — ppm from tetramethylsilane (TMS) as reference.

(b) Similarly, proceeding as is described in Subpart (a) above of this Example using HPLC separation there is obtained:

N-acetyl-O,O,O,N,H,N-pentaoctanoyl deferoxamine; and
N-acetyl-O,O,O,N,H,N-hexaoctanoyl deferoxamine.

Example 2

(1) Preparation of N-Octanoyl-O,O,O-trioctanoyldeferoxamine
(Step 1, Schotten-Baumann conditions)

(a) Deferoxamine mesylate (6.0 g) is suspended in 50 ml of water and 50 ml of dioxane. The suspension is adjusted to pH of 9 using 5N sodium hydroxide solution with strong agitation. In 10 ml portions, a solution of 13.0 g of octanoyl choride in 60 ml of dioxane. The pH of the mixture is maintained at 9 by the dropwise addition of the 5N sodium hydroxide solution. After the addition 40 ml of the octanoyl chloride/dioxane solution, the reaction mixture is treated with 50 ml of water and 200 ml of chloforom. The mixture separates into two phases which are agitated strongly. After the addition of all the acid chloride solution, the reaction mixture is agitated for 2 hr at pH of 9. The reaction mixture is diluted with 500 ml of water and 1000 ml of chloroform and the aqueous phase is separated and extracted twice using 250 ml portions of chloroform. The combined chloroform phases are washed twice with saturated sodium bicarbonate solution, twice with saturated sodium chloride solution and dried using anhydrous sodium sulfate. The chloroform is removed under reduced pressure, and the waxy residue is dissolved in 500 ml of diethyl ether and hexane was added almost to turbidity. A sticky, granular product weighing 3.8 g is obtained. The infrared and proton magnetic resonance spectra are consistent with a structure of N-octanoyl-O,O,O-trioctanoyldeferoxamine.

(b) Similarly, proceeding as in Subpart (a) above, but substituting a stoichiometrically equivalent amount of

acetyl chloride;
propionyl chloride;
butyryl chloride;
pivalyl chloride;
valeryl chloride;
isovaleryl chloride;
dodecanoyl chloride;
palmitoyl chloride or
hexacosanoyl chloride;

instead of octanoyl chloride, there is obtained the corresponding

N-acetyl-O,O,O-triacetyldeferoxamine;
N-propionyl-O,O,O-tripropionyldeferoxamine;

N-butyryl-O,O,O-tributyryldeferoxamine;
N-pivalyl-O,O,O-tripivalyldeferoxamine;
N-valeryl-O,O,O-trivaleryldeferoxamine;
N-isovaleryl-O,O,O-triisovaleryldeferoxamine;
N-dodecanoyl-O,O,O-tridodecanoyldeferoxamine;
N-palmitoyl-O,O,O-tripalmitoyldeferoxamine;
or
N-hexacosanoyl-O,O,O-trihexacosanoyldeferoxamine.

(c) Similarly, proceeding as in Subpart (a) above, but substituting a stoichiometrically equivalent amount of
3-chlorobutanoyl chloride;
3-chloroisovaleryl chloride;
10-chlorooctadecanoyl chloride;
10-methoxyoctadecanoyl chloride;
9,10-dichlorooctadecanoyl chloride;
9,10-dibromooctadecanoyl chloride; or
9,10-dimethoxyoctadecanoyl chloride;
instead of octanoyl chloride, there is obtained the corresponding
N-(3-chlorobutanoyl)-O,O,O-tri(3-chlorobutanoyl)deferoxamine;
N-(3-chloroisovaleryl)-O,O,O-tri(3-chloroisovaleryl)deferoxamine;
N-(10-chlorooctadecanoyl)-O,O,O-tri(10-chlorooctadecanoyl)deferoxamine;
N-(10-methoxyoctadecanoyl)-O,O,O-tri(10-methoxyoctadecanoyl)deferoxamine;
N-(9,10-dichlorooctadecanoyl)-O,O,O-tri(9,10-dichlorooctadecanoyl)deferoxamine;
N-(9,10-dibromooctadecanoyl)-O,O,O-tri(9,10-dibromooctadecanoyl)deferoxamine; or
N-(9,10-dimethoxyoctadecanoyl)-O,O,O-tri(9,10-dimethoxyoctadecanoyl)deferoxamine.
N-cyclopropylacetyl-O,O,O-tri(cyclopropylacetyl)deferoxamine;
N-cyclobutylacetyl-O,O,O-tri(cyclobutylacetyl)deferoxamine;
N-cyclohexylpropanoyl-O,O,O-tri(cyclohexylpropanoyl)deferoxamine;
N-cyclodecyldecanoyl-O,O,O-tri(cyclodecyldecanoyl)deferoxamine; or
N-cyclopentacosanylacetyl-O,O,O-tri(cyclopentacosanylacetyl)deferoxamine.

(d) Similarly proceeding as in Subpart (a) above, but substituting a stoichiometrically equivalent amount of 10-(2-chloropentacosanyldecanoyl chloride instead of octanoyl chloride, there is obtained the corresponding
N-[10-(2-chloropentacosanyl)decanoyl]-O,O,O-tri[10-(2-chloropentacosanyl)decanoyl]deferoxamine.

(2) Preparation of N-Octanoyl-O,O,O-trihydrogendeferoxamine (Step 2)

(a) N-Octanoyl-O,O,O-trioctanoyldeferoxamine (3.5 g, from Example 2 (1)) is dissolved in 250 ml of ether, 100 ml of methanol and saturated at ambient temperature with ammonia gas. After stirring for 3 days (72 hr) at ambient temperature, the reaction mixture is evaporated to dryness using reduced pressure, and the solid residue is boiled five times with hexane to remove the octanoic acid amide. The remaining colorless crystalline product is recrystallized from n-propanol/water (66/34) to give 1.5 g of N-octanoyl-O,O,O-trihydrogendeferoxamine, m.p. 185—187°. The infrared and nuclear magnetic resonance spectra are consistent with this structure. The product is sparingly soluble in water and ordinary organic solvents.

(b) Similarly, the proceeding as is described above in Subpart (a) of this Example, but substituting a stoichiometrically equivalent amount of the deferoxamines as prepared in Example 2(1), Subpart (b) above for N-octanoyl-O,O,O-trioctanoyldeferoxamine, there is obtained the corresponding
N-acetyl-O,O,O-trihydrogendeferoxamine;
N-propionyl-O,O,O-trihydrogendeferoxamine;
N-butyryl-O,O,O-trihydrogendeferoxamine;
N-pivalyl-O,O,O-trihydrogendeferoxamine;
N-valeryl-O,O,O-trihydrogendeferoxamine;
N-isovaleryl-O,O,O-trihydrogendeferoxamine;
M-dodecanoyl-O,O,O-trihydrogendeferoxamine;
N-palmitoyl-O,O,O-trihydrogendeferoxamine;
or
N-hexacosanoyl-O,O,O-trihydrogendeferoxamine.

(c) Similarly, proceeding as is described above in Subpart (a) of this Example, but substituting a stoichiometrically equivalent amount of the deferoxamines as prepared in Example 2(1), Subpart (c) above for N-octanoyl-O,O,O-trioctanoyldeferoxamine, there is obtained the corresponding
N-(3-chlorobutanoyl)-O,O,O-trihydrogendeferoxamine;
N-(3-chloroisovaleryl)-O,O,O-trihydrogendeferoxamine;
N-(10-chlorooctadecanoyl)-O,O,O-trihydrogendeferoxamine;
N-(10-methoxyoctadecanoyl)-O,O,O-trihydrogendeferoxamine;
N-(9,10-dichlorooctadecanoyl)-O,O,O-trihydrogendeferoxamine;
N-(9,10-dibromooctadecanoyl)-O,O,O-trihydrogendeferoxamine; or

N-(9,10-dimethoxyoctadecanoyl)-O,O,O-trihydrogendeferoxamine.

(d) Similarly, proceeding as is described above in Subpart (a) of this Example, but substituting a stoichiometrically equivalent amount of the deferoxamine as prepared in Example 2(1), Subpart (d) above for N-octanoyl-O,O,O-trioctanoyldeferoxamine, there is obtained the corresponding

N-[10-(2-chloropentacosanyl)decanoyl]-O,O,O-trihydrogendeferoxamine.

(3) Preparation of N-Octanoyl-O,O,O-tributyryldeferoxamine (Step 3)

(a) N-Octanoyl-O,O,O-trihydrogen deferoxamine [1.5 g, from Example 2(2) (a)] is suspended in a solution of 50 ml of water and 50 ml of chloroform. The well-agitated suspension is adjusted to pH of 9 using 5N sodium hydroxide solution. To this mixture is added dropwise, a solution of 1.4 g of butyryl chloride in 30 ml of chloroform. The pH of 9 of the mixture is maintained by the addition of a 5N sodium hydroxide solution as needed. After 20 ml of the butyryl chloride solution are added 25 ml of water and 100 ml of chloroform are added to facilitate the mixing of the solution. After the butyryl chloride solution is all added, the reaction mixture is stirred for 2 hours, with periodic adjustment to maintain a pH of 9. The reaction mixture is then diluted with 50 ml of water and 200 ml of chloroform and centrifuged to separate the phases. Any white solid at the interface is removed and discarded. The chloroform phase is washed twice with 100 ml of saturated sodium bicarbonate solution and twice with 100 ml of saturated sodium chloride solution, dried using anhydrous sodium sulfate, filtered and reduced to dryness using reduced pressure. 2.0 g of a crude waxy white solid is obtained, which is washed twice with ether and recrystallized form 60% ethanol. The solid is air dried to produce 1.1 g of N-octanoyl-O,O,O-tributyryldeferoxamine. The infrared spectra and nuclear magnetic resonance spectra are consistent with this structure.

(b) Similarly, proceeding as is described above in Subpart (a) of this Example, but substituting a stoichiometrically equivalent amount of the deferoxamines as prepared in Example 2(2), Subpart (b) above for N-octanoyl-O,O,O-trihydrogendeferoxamine, there is obtained the corresponding

N-acetyl-O,O,O-tributyryldeferoxamine;
N-propionyl-O,O,O-tributyryldeferoxamine;
N-butyryl-O,O,O-tributyryldeferoxamine;
N-pivalyl-O,O,O-tributyryldeferoxamine;
N-valeryl-O,O,O-tributyryldeferoxamine;
N-isovaleryl-O,O,O-tributyryldeferoxamine;
N-dodecanoyl-O,O,O-tributyryldeferoxamine;
N-palmitoyl-O,O,O-tributyryldeferoxamine; or
N-hexacosanoyl-O,O,O-tributyryldeferoxamine.

(c) Similarly, proceeding as is described above in Subpart (a) of this Example, but substituting a stoichiometrically equivalent amount of the deferoxamines as prepared in Example 2(2), Supbart (c) above for N-octanoyl-O,O,O-trihydrogendeferoxamine, there is obtained the corresponding

N-(3-chlorobutanoyl)-O,O,O-tributyryldeferoxamine;
N-(3-chloroisovaleryl)-O,O,O-tributyryldeferoxamine;
N-(10-chlorooctadecanoyl)-O,O,O-tributyryldeferoxamine;
N-(10-methoxyoctadecanoyl)-O,O,O-tributyryldeferoxamine;
N-(9,10-dichlorooctadecanoyl)-O,O,O-tributyryldeferoxamine;
N-(9,10-dibromooctadecanoyl)-O,O,O-tributyryldeferoxamine; or
N-(9,10-dimethoxyoctadecanoyl)-O,O,O-tributyryldeferoxamine.

(d) Similarly, proceeding as is described above in Subpart (a) of this Example, but substituting a stoichiometrically equivalent amount of the deferoxamine as prepared in Example 2(2), Subpart (d) above for N-octanoyl-O,O,O-trihydrogendeferoxamine, there is obtained the corresponding

N-(2-chloropentadecylacetyl)-O,O,O-tributyldeferoxamine; or
N-[10-(2-chloropentacosanyl)decanoyl]-O,O,O-tributyryldeferoxamine.

(e) Similarly, proceeding as is described above in Subpart (a) of this Example, but substituting one half of the stoichiometrically equivalent amount of the following equimolar mixture of acyl chlorides:

butyryl chloride and octanoyl chloride for butyryl chloride, there is obtained a corresponding mixture of products, including:

N-octanoyl-O,O,O-butyryloctanoylhydrogendeferoxamine; N-octanoyl-O,O,O-octanoylhydrogenbutyryldeferoxamine;
or
N-octanoyl-O,O,O-hydrogenbutyryldeferoxamine.

The exact positions of the acyl or hydrogen groups has not yet been established with certainty.

In Examples 3 and 4, the active ingredient is N-acetyl-O,O,O-trioctanoyldeferoxamine.

Example 3
Tablet Formation

| Ingredients | Quantity Tablet, mgs. |
|---|---|
| Active Ingredient | 350 |
| Cornstarch | 20 |
| Lactose, spray dried | 100 |
| Magnesium stearate | 2 |

The above ingredients are thoroughly mixed, granulated, and pressed into single scored tablets.

Example 4
Capsule Formation

| Ingredients | Quantity Capsule, mgs. |
|---|---|
| Active Ingredient | 350 |
| Lactose, spray dried | 100 |
| Magnesium stearate | 2 |

The above ingredients are mixed and introduced into a hard-shell gelatin capsule.

**Claims**

1. A compound of the formula:

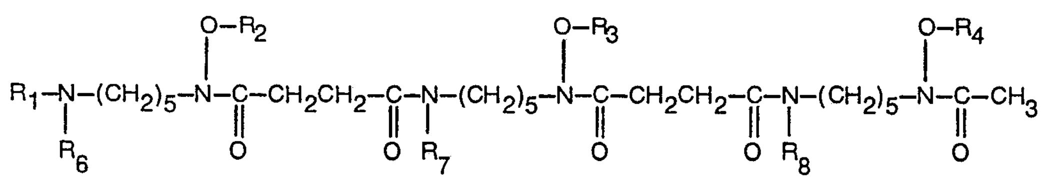

(I)

wherein:

$R_1$ is acyl of the formula —(C=O)—$R_5$; and

$R_2$, $R_3$, $R_4$, $R_6$, $R_7$ and $R_8$ are each independently selected from hydrogen, and acyl of the formula:

$$—C(=O)—R_5$$

wherein $R_5$ is selected from $C_{1-25}$ alkyls and substituted $C_1$—$C_{25}$ alkyls having 1 to 3 protons replaced by alkoxyl or halogen; such that at least one $R_2$, $R_3$ and $R_4$ is an acyl of the formula —C(=O)—$R_5$ different than $R_1$, when $R_6$, $R_7$ and $R_8$ are each hydrogen.

2. The compound of Claim 1 wherein in $R_1$ the alkyl groups are $C_{1-7}$ alkyl groups and $R_2$, $R_3$, $R_4$, $R_6$, $R_7$ and $R_8$ are independently selected from hydrogen and acyl wherein $R_5$ is $C_{1-7}$ alkyl.

3. The compound of Claim 1 or 2 wherein $R_6$, $R_7$ and $R_8$ are each hydrogen.

4. The compound of any of Claims 1 to 3 wherein $R_1$ is acetyl.

5. The compound of Claim 4 wherein $R_2$, $R_3$ and $R_4$ are identical.

6. The compound of Claim 5 wherein $R_1$ is acetyl and $R_2$, $R_3$ and $R_4$, $R_5$ is n-heptyl.

7. The compound of Claim 6 wherein $R_1$ is acetyl; $R_2$, $R_3$ and $R_4$ are each acyl wherein $R_5$ is n-heptyl; and two of $R_6$, $R_7$ and $R_8$ are hydrogens, and one of the remaining $R_6$, $R_7$ or $R_8$ is an acyl where $R_5$ is n-heptyl.

8. The compound of any of Claims 1 to 3, wherein in $R_1$, $R_5$ is $C_{1-7}$ alkyl, and in each of $R_2$, $R_3$ nd $R_4$, $R_5$ is $C_{8-15}$ alkyl.

9. The compound of Claim 8 wherein in $R_1$, $R_5$ is ethyl and in each of $R_2$, $R_3$ and $R_4$, $R_5$ is nonyl.

10. A pharmaceutical composition for treating an ion overload condition in a human being which comprises a therapeutically effective amount of a compound as defined in any of claims 1 to 9.

11. The composition of claim 10, wherein $R_2$, $R_3$ and $R_4$ are each identical acyl groups.

12. The composition of claim 11, wherein said identical acyl groups are selected from acyls, wherein $R_5$ is selected from ethyl, n-propyl, n-butyl, i-butyl, t-butyl and n-pentyl.

13. A process for the preparation of acyl substituted deferoxamines represented by the following formula:

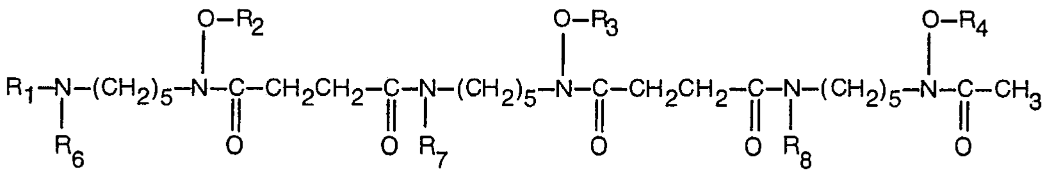

(I)

wherein:

$R_1$ is acyl of the formula —(C=O)—$R_5$;

$R_2$, $R_3$, $R_4$, $R_6$, $R_7$ and $R_8$ are independently selected from hydrogen and acyl of the formula —C(=O)—$R_5$, wherein $R_5$ is selected from $C_{1-25}$ alkyls and substituted $C_1$—$C_{25}$ alkyls having 1 to 3 protons replaced by alkoxyl or halogen; such that at least one $R_2$, $R_3$ and $R_4$ is an acyl of the formula —C(=O)—$R_5$; which process comprises:

(a) contacting the unsubstituted deferoxamine, wherein $R_1$, $R_2$, $R_3$, $R_4$, $R_6$, $R_7$ and $R_8$ are each hydrogen, with a suitable acylating agent in the presence of a strong base;

(b) treating the product of step (a) with a weak base to form the N-acyl-O,O,O-trihydrogendeferoxamine; and

(c) treating the product of step (b) with one or more different acylating agents to form the compound of formula I.

14. The process of Claim 13 wherein:

(a) in step (a) said suitable acylating agent is $R_5$—(C=O)OC(=O)—$R_5$; wherein $R_5$ contains 1 to 7 carbon atoms;

(b) in step (b) said weak base has a $pK_b$ of 4 to 6; and

(c) in step (c) said different acylating agent is ($R_5$C(=O)—X wherein $R_5$ contains 1 to 7 carbon atoms and X is halogen.

15. The process of Claim 14 wherein:

(a) in step (a) said acylating agent is acetic anhydride;

(b) in step (b) said base is gaseous anhydrous ammonia; and

(c) in step (c) said different acylating agent is octanoyl chloride.

16. Use of a compound of any of claims 1 to 9 for the manufacture of a medicament for treating ion overload conditions.

**Patentansprüche**

1. Verbindung der Formel

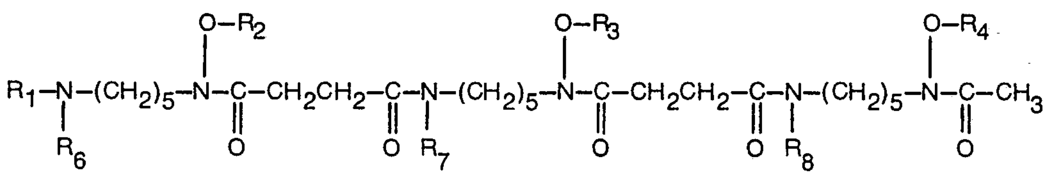

(I)

worin

$R_1$ Acyl der Formel —(C=O)—$R_5$ ist; und

$R_2$, $R_3$, $R_4$, $R_6$, $R_7$ und $R_8$ jeweils unabhängig aus Wasserstoff und Acyl der Formel ausgewählt sind

—C(=O)—$R_5$

worin

$R_5$ aus $C_{1-25}$-Alkylen und substituierten $C_1$—$C_{25}$-Alkylen, bei denen 1 bis 3 Protonen durch Alkoxyl oder Halogen ersetzt sind, ausgewählt ist; wobei wenigstens ein $R_2$, $R_3$ und $R_4$ eine von $R_1$ verschiedenes Acyl der Formel —C(=O)—$R_5$ ist, wenn $R_6$, $R_7$ und $R_8$ jeweils Wasserstoff sind.

2. Verbindung nach Anspruch 1, worin die Alkylgruppen von $R_1$ $C_1$—$C_7$-Alkylgruppen sind und $R_2$, $R_3$, $R_4$, $R_6$, $R_7$ und $R_8$ unabhängig aus Wasserstoff und Acyl, worin $R_5$ $C_1$—$C_7$-Alkyl ist, ausgewählt sind.

3. Verbindung nach Anspruch 1 oder 2, worin $R_6$, $R_7$ und $R_8$ jeweils Wasserstoff sind.

4. Verbindung nach einem der Ansprüche 1 bis 3, worin $R_3$ Acetyl ist.

5. Verbindung nach Anspruch 4, worin $R_2$, $R_3$ und $R_4$ identisch sind.

6. Verbindung nach Anspruch 5, worin $R_1$ Acetyl ist und in $R_2$, $R_3$ und $R_4$, $R_5$ n-Heptyl ist.

7. Verbindung nach Anspruch 6, worin, $R_1$ Acetyl ist; $R_2$, $R_3$ und $R_4$ jeweils Acyl sind, worin $R_5$ n-Heptyl

ist; und zwei von $R_6$, $R_7$ und $R_8$ Wasserstoffe sind sowie eines der verbleibenden $R_6$, $R_7$ oder $R_8$ ein Acyl ist, worin $R_5$ n-Heptyl ist.

8. Verbindung nach einem der Ansprüche 1 bis 3, worin in $R_1$, $R_5$ $C_{1-7}$-Alkyl ist und in jedem von $R_2$, $R_3$ und $R_4$, $R_5$ $C_{8-15}$-Alkyl ist.

9. Verbindung nach Anspruch 8, worin in $R_1$, $R_5$ Ethyl ist und in jedem von $R_2$, $R_3$ und $R_4$, $R_5$ Nonyl ist.

10. Pharmazeutische Zusammensetzung zur Behandlung eines Ionen-Überbeladungszustands im Menschen, welche eine therapeutische wirksame Menge einer Verbindung, wie in einem der Ansprüche 1 bis 9 definiert, umfaßt.

11. Zusammensetzung nach Anspruch 10, worin $R_2$, $R_3$ und $R_4$ jeweils identische Acylgruppen sind.

12. Zusammensetzung nach Anspruch 11, worin die identischen Acylgruppen aus Acylen ausgewählt sind, in denen $R_5$ aus Ethyl, n-Propyl, n-Butyl, i-Butyl, t-Butyl and n-Pentyl ausgewählt sind.

13. Verfahren zur Herstellung von Acyl-substitutierten Deferoxaminen, die durch die folgende Formel wiedergegeben werden

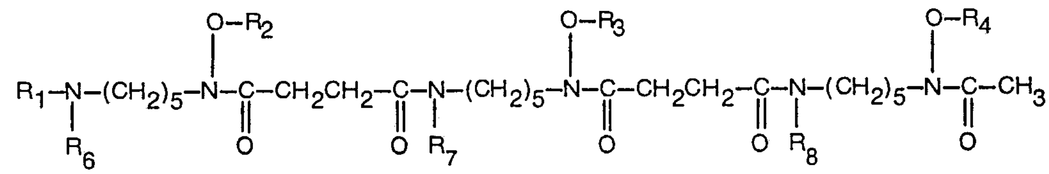

(I)

worin

$R_1$ Acyl der Formel —C(=O)—$R_5$ ist;

$R_2$, $R_3$, $R_4$, $R_6$, $R_7$ und $R_8$ unabhängig aus Wasserstoff und Acyl der Formel —C(=O)—$R_5$ ausgewählt sind,

worin

$R_5$ aus $C_{1-25}$-Alkylen und substituierten $C_1$—$_{25}$-Alkylen, bei denen 1 bis 3 Protonen durch Alkoxyl oder Halogen ersetzt sind, ausgewählt ist; wobei wenigstens eines $R_2$, $R_3$ und $R_4$ Acyl der Formel —C(=O)—$R_5$ ist;

welches Verfahren umfaßt

(a) das Inberührungsbringen von unsubstituiertem Deferoxamin, worin $R_1$, $R_2$, $R_3$, $R_4$, $R_6$, $R_7$ und $R_8$ jeweils Wasserstoff sind, mit einem geeigneten Acylierungsmittel in Gegenwart einer starken Base;

(b) Behandeln des Produkts aus Schritt (a) mit einer schwachen Base zur Bildung von N-Acyl-O,O,O-trihydrogendeferoxamin; und

(c) Behandeln des Produkts aus Schritt (b) mit einem oder mehreren verschiedenen Acylierungsmitteln unter Bildung der Verbindung der Formel I.

14. Verfahren nach Anspruch 13, worin

(a) in Schritt (a) das geeignete Acylierungsmittel $R_5$—(C=O)—OC(=O)—$R_5$ ist, worin $R_5$ 1 bis 7 Kohlenstoffatome enthält;

(b) in Schritt (b) die schwache Base eine $pK_b$ von 4 bis 6 hat; und

(c) in Schritt (c) das verschiedene Acylierungsmittel $R_5C$(=O)—X ist, worin, $R_5$ 1 bis 7 Kohlenstoffatome enthält und X Halogen ist.

15. Verfahren nach Anspruch 14, worin

(a) in Schritt (a) das Acylierungsmittel Essigsäureanhydrid ist;

(b) in Schritt (b) die Base gasförmige wasserfreies Ammoniak ist; und

(c) in Schritt (c) das verschiedene Acylierungsmittel Octanoylchlorid ist.

16. Verwendung einer Verbindung nach einem der Ansprüche 1 bis 9 zur Herstellung eines Medikamments zur Behandlung von Ionen-Überbeladungszuständen.

**Revendications**

1. Une composé de formule:

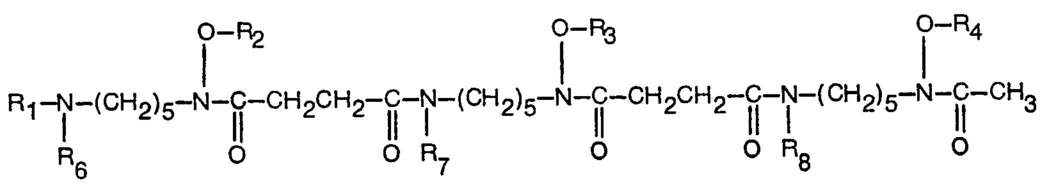

(I)

dans lequel:

$R_1$ est un acyle de formule —(C=O)—$R_5$; et

$R_2$, $R_3$, $R_4$, $R_6$, $R_7$ et $R_8$ sont choisis chacun indépendamment parmi l'hydrogène et l'acyle de formule:

—C(=O)—$R_5$

$R_5$ étant choisi parmi les alcoyles $C_{1-25}$ et les alcoyles substitués $C_{1-25}$ dans lesquels 1 à 3 protons ont été remplacés par un alcoyle par un halogène; ainsi en fin de compte un $R_2$, $R_3$ et $R_4$ est un acyle de formule —C(=O)—$R_5$ différent de $R_1$, $R_6$, $R_7$ et $R_8$ étant constitués chacun par de l'hydrogène.

2. Le composé de la revendication 1 dans lequel les groupes alcoyles de $R_1$ sont des groupes alcoyles $C_{1-7}$ et $R_2$, $R_3$, $R_4$, $R_6$, $R_7$ et $R_8$ sont choisis indépendamment parmi l'hydrogène et l'acyle, $R_5$ étant un alcoyle $C_{1-7}$.

3. Le composé de la revendication 1 ou 2 dans lequel $R_6$, $R_7$ et $R_8$ sont constitués chacun par de l'hydrogène.

4. Le composé de l'une des revendications 1 à 3 dans lequel $R_1$ est un acétyle.

5. Le composé de la revendication 4 dans lequel $R_2$, $R_3$ et $R_4$ sont identiques.

6. Le composé de la revendication 5 dans lequel $R_1$ est un acétyle et/eu $R_2$, $R_3$ et $R_4$, $R_5$ renferme du n-heptyle.

7. Le composé de la revendication 6 dans lequel $R_1$ est un acétyle;

$R_2$, $R_3$ et $R_4$ sont constitués chacun d'un acyle dans lequel $R_5$ est un n-heptyle; et deux de $R_6$, $R_7$ et $R_8$ sont des hydrogènes et l'un des $R_6$, $R_7$ et $R_8$ restants est un acyle dans lequel $R_5$ est un n-heptyle.

8. Le composé de l'une revendications 1 à 3 dans lequel $R_1$, $R_5$ est un alcoyle $C_{1-7}$ et/eu chacun des $R_2$, $R_3$ et $R_4$, $R_5$ referme un alcoyle $C_{8-15}$.

9. Le composé de la revendication 8 dans lequel eu $R_1$, $R_5$ renferme un éthyle et/eu $R_2$, $R_3$ et $R_4$, $R_5$ est du nonyle.

10. Un composé pharmaceutique pour traiter un état de surcharge en ions affectant une personne et dont la valeur thérapeutique effective est celle définie dans l'une des revendications 1 à 9.

11. Le composé de la revendication 10 dans lequel $R_2$, $R_3$ et $R_4$ représentent chacun des groupe alcyls identiques.

12. Le composé de la revendication 11 dans lequel les dits groupes acyles identiques sont choisis parmi des acyles où $R_5$ est choisis parmi l'éthyle, le n-propyle, le n-butyle, le i-butyle, le t-butyle et le n-pentyle.

13. Une procédé pour la préparation de déféroxamines substituées par un acyle que représente la formule suivante:

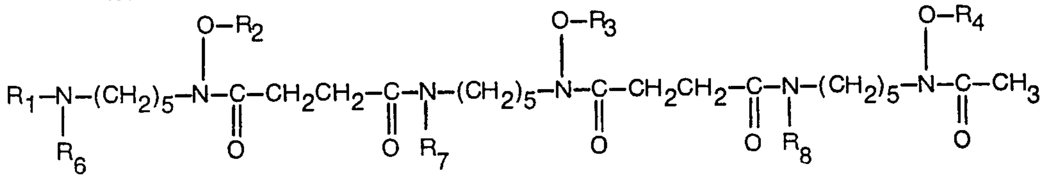

(I)

où:

$R_1$ est un acyle de formule —(C=O)—$R_5$;

$R_2$, $R_3$, $R_4$, $R_6$, $R_7$ et $R_8$ sont choisis indépendamment parmi l'hydrogene et l'acyle de formule —C(=O)—$R_5$, $R_5$ étant choisi parmi les alcoyles $C_{1-25}$ et les alcoyles substitués $C_{1-25}$ dans lesquels 1 à 3 protons ont été remplacés par un alcoyle par un halogène, de sorte qu'à la fin l'un des $R_2$, $R_3$ et $R_4$ est un acyle de formule —(C=O)—$R_5$;

le procédé comprenant:

(a) une mise en contact de la déféroxamine non substituée, dans laquelle $R_1$, $R_2$, $R_3$, $R_4$, $R_6$, $R_7$ et $R_8$ sont constitués chacun par de l'hydrogène, avec un agent acylant convenable et en présence d'une base forte;

(b) le traitement du produit de l'étape (a) par une base faible pour former la N-acyl-O,O,O-trihydrogène-déféroxamine; et

(c) le traitement du produit de l'étape (b) par un ou plusieurs agents acylants différents pour former le composé de formule I.

14. Le procédé de la revendication 13 dans lequel:

(a) dans l'étape (a) le dit agent acylant convenable est $R_5$—(C=O)OC(=O)—$R_5$; dans lequel $R_5$ renferme de 1 à 7 atomes de carbone;

(b) dans l'étape (b) la dite base faible a un $pK_b$ de 4 à 6; et

(c) dans l'étape (c) de dit agent acylant différent est $R_5C$(=O)—X dans lequel $R_5$ renferme de 1 à 7 atomes de carbone et X est halogène.

15. Le procédé de la revendication 14 dans lequel:

(a) dans l'étape (a) le dit agent acylant est de l'anhydride acétique;

(b) dans l'étape (b) la dite base est le gaz ammoniac anhydre;

(c) dans l'étape (c) de dit agent acylant différent est du chlorure d'octanoyle.

16. Emploi d'un composé précisé dans l'une des revendications 1 à 9 pour la fabrication d'un médicament en vue du traitement des états de surcharge en ions.